# FASCICULE DE BREVET EUROPEEN

(11) **EP 0 787 205 B1**
(45) Date de publication et mention de la délivrance du brevet: **12.08.1998**
(21) Numéro de dépôt: 95936616.2
(22) Date de dépôt: 27.10.1995
(51) Int. Cl.: C12Q 1/68

(54) **Procédé d'amplification d'acides nucléiques en phase solide et trousse de réactifs pour sa mise en oeuvre**
Verfahren zur Amplifikation von Nukleinsäuren auf einer Festphase und Reagenziensatz dafür
Solid-phase nucleic acid amplification method and reagent kit therefor

(30) Priorité: 28.10.1994 FR 9412972
(43) Date de publication de la demande: 06.08.1997
(73) Titulaire: GENSET, 75011 Paris (FR)
(72) Inventeur: PEPONNET, Christine, F-75010 Paris (FR)
(74) Mandataire: Warcoin, Jacques
(86) Numéro de dépôt international: FR9501422
(87) Numéro de publication internationale: WO9613609

(56) Documents cités:
- EP-A- 0 480 408
- WO-A-90/01546
- WO-A-91/00868
- WO-A-93/03052
- WO-A-93/04199
- WO-A-93/09250
- WO-A-93/13220
- WO-A-93/15228
- CLINICAL CHEMISTRY, vol.40, no.2, 1994, page 200 - 205, ''

## Description

La présente invention concerne un procédé d'amplification d'acides nucléiques en phase solide, ainsi qu'une trousse de réactifs utile pour la mise en oeuvre du procédé.

La présente invention a également pour objet un procédé d'immobilisation d'une amorce sur phase solide.

L'amplification sur une phase solide consiste en l'élongation, au cours d'une réaction de PCR ou d'autres types d'amplifications, telle que LCR, SDA etc... d'une amorce préalablement fixée à un support solide. Une telle technique permet d'obtenir un produit d'amplification dont un brin spécifique est attaché de façon covalente à la phase solide, sans avoir recours à d'autres étapes que la PCR. Ceci permet d'effectuer la détection avant dénaturation sur de l'ADN double brin, ou après dénaturation sur un brin spécifique du produit d'amplification en enchaînant PCR et détection sans changer de support.

Des méthodes d'amplification d'acides nucléiques en phase solide ont été décrites dans WO 89/11546, AU 47144/89 et WO 93/09250.

Ce type d'amplification sur support peut être très utile pour toutes les applications de diagnostic en biologie moléculaire, notamment pour la détection de cibles infectieuses ou de cibles génomiques. Cette technique permet de réduire les temps de détection de même que les risques d'erreurs, puisque l'échantillon n'est pas transféré d'un puits à un autre mais que toute l'expérience se passe sur le même support. Par ailleurs, pour toutes les applications ne nécessitant qu'un seul brin spécifique d'ADN, comme le clonage ou le séquençage, cette technique peut permettre un réel gain de temps et une grande facilité d'utilisation évitant beaucoup d'étapes intermédiaires.

L'amorce participant à la PCR fixée par son extrémité 5' au support solide doit faire partie du brin que l'on souhaite allonger sur le support solide. L'extrémité 3' de l'amorce doit être libre, non modifiée et homologue à la cible pour permettre son extension par une polymérase.

Toutefois, un inconvénient majeur de l'amplification en phase solide est le faible rendement de l'élongation sur la phase solide.

Afin de diminuer les interactions stériques support solide-oligonucléotides et d'améliorer l'accessibilité de la Taq polymérase à l'hybride formé par l'amorce fixée et le produit d'amplification complémentaire, un bras de liaison ou "linker" est placé à l'extrémité 5' de l'amorce fixée. Ce bras de liaison est aussi appelé "bras espaceur" car il sert à éloigner physiquement du support solide l'extrémité 3' de l'amorce fixée pour ne pas gêner la coopération des différents réactifs d'amplification avec l'amorce.

On a découvert, selon la présente invention, que l'un des paramètres influençant le rendement d'élongation, réside dans le mode de fixation de l'amorce sur le support solide. Les nucléotides de l'amorce eux-mêmes sont susceptibles d'être impliqués dans une fixation covalente avec le support dans les conditions de couplage utilisées entre le "linker" et le support solide, ce qui contribue au faible rendement d'élongation de l'amorce. Plus précisément, on a découvert que plus que la taille du bras de liaison, c'est la réactivité ou la mutiplicité des sites potentiels de fixation du bras de liaison sur le support qui augmente le rendement d'élongation de l'amorce.

La présente invention a pour objet un procédé d'amplification d'acides nucléiques en phase solide dans lequel on utilise une amorce immobilisée sur un support solide thermo-résistant fonctionnalisé, caractérisé en ce que ladite amorce est immobilisée sur le support solide par l'intermédiaire d'un lien covalent entre le support solide et un groupe fonctionnel d'une molécule polyfonctionnelle, ladite molécule étant elle-même liée à l'extrémité 5' de ladite amorce.

Plus précisément ladite amorce est immobilisée sur le support solide par l'intermédiaire d'un bras de liaison (ou "linker") consistant en un reste de ladite molécule polyfonctionnelle intercalé entre le support solide et l'extrémité 5' de l'amorce et établissant un lien covalent entre un groupe fonctionnel du support solide et un premier groupe fonctionnel de ladite molécule polyfonctionnelle, d'une part, et entre l'extrémité 5' de l'amorce et un deuxième groupe fonctionnel de ladite molécule polyfonctionnelle d'autre part.

En augmentant le nombre de groupes fonctionnels dans le bras de liaison on augmente le rendement d'élongation. On pense que cela est dû au fait que l'on augmente ainsi la probabilité que la fixation sur le support solide se fasse par l'intermédiaire du bras de liaison, c'est-à-dire sans que l'amorce proprement dite, soit elle-même impliquée.

On entend ici par groupe fonctionnel de ladite molécule polyfonctionnelle, un groupe susceptible d'établir un lien covalent avec le support solide. On cite en particulier, comme groupes fonctionnels, les groupes amine, hydroxyl, carboxyl, aldehyde, thiol ou phosphate.

Lorsque le bras de liaison comporte un groupe très réactif tel qu'un groupe phosphate terminal à son extrémité non liée à l'amorce, il n'est pas nécessaire que la molécule polyfonctionnelle comporte un nombre élevé de groupes fonctionnels. Toutefois, de préférence ladite molécule polyfonctionnelle comporte au moins cinq, de préférence encore, au moins dix groupes fonctionnels.

Dans un mode de réalisation avantageux de l'invention, ladite molécule polyfonctionnelle comprend un fragment polymère. De préférence chaque unité monomérique dudit fragment polymère comporte au moins un groupe fonctionnel. Dans ce cas l'augmentation de la taille du bras de liaison, c'est-à-dire du polymère, se traduit par l'augmentation du nombre de sites potentiels de fixation terminale de l'amorce et permet d'augmenter le rendement d'élongation et ce, alors que le taux global de fixation de l'amorce sur le support solide reste constant.

De préférence le fragment polymère rajouté à l'extrémité 5' de ladite amorce comporte plus de 5 monomères de préférence encore plus de 10 monomères, notamment jusqu'à 50 monomères.

Avantageusement ledit fragment polymère selon l'invention, comprend un fragment homopolynucléotidique ou un fragment d'un analogue de polynucléotides.

On entend ici par "analogue de polynucléotides" un polymère dont les unités monomériques sont reliées par un lien phosphodiester semblable au lien phosphodiester des polynucléotides naturels. En d'autres termes, il s'agit d'un polymère dans lequel les restes nucléosidiques des monomères nucléotidiques sont remplacés par des restes non nucléosidiques, notamment aliphatiques. On cite en particulier des fragments polymères de formule (I) suivante : 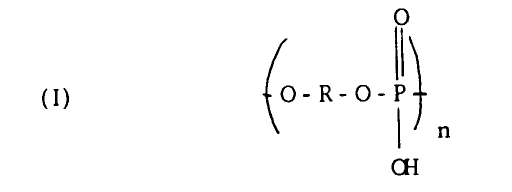 dans laquelle R est un reste aliphatique, notamment de 2 à 20 atomes de carbone, tel qu'un reste alkylène, R comportant au moins un groupe fonctionnel selon l'invention et n est un entier notamment de 2 à 50.

De préférence le fragment polymère de type analogue de polynucléotides selon l'invention comporte plusieurs groupes réactifs greffés sur chaque monomère, en particulier des groupes amine ou hydroxyl, dans ce cas n peut être plus particulièrement compris entre 2 et 10 seulement.

Lorsque le bras de liaison consiste en un fragment homopolynucléotide, il comporte de préférence plus de 5 nucléotides, de préférence encore plus de 10 nucléotides notamment jusqu'à 50 nucléotides.

Les analogues de polynucléotides selon l'invention, peuvent être obtenus par des méthodes de synthèse similaires aux méthodes de synthèse d'ADN, notamment par synthèse automatique sur support solide. En effet, ces fragments polymères peuvent être obtenus par condensation des synthons monomériques adaptés aux procédés de synthèse nucléotidique impliquant l'utilisation de synthons phosphoramidites, c'est-à-dire un synthon comportant de façon classique deux groupes OH terminaux, protégés, l'un par un groupe Diméthoxytrityl (Dmtr) et l'autre par un groupe phosphoramidite. Le fragment polymère de type analogue de polynucléotide correspond alors à la condensation de synthons similaires aux synthons nucléotidiques classiques dans lesquels le reste nucléosidique divalent en 5' et 3', est remplacé par un reste aliphatique, notamment alkylène.

Plus particulièrement, pour obtenir les fragments de polymères analogues de polynucléotides de formule (I), on peut utiliser des synthons phosphoramidites de formule :

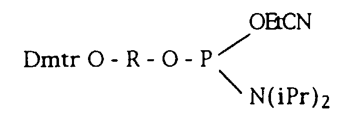

Ainsi lorsque le bras de liaison est un fragment polynucléotide ou analogue de polynucléotide, on peut avantageusement préparer directement par synthèse de type nucléotidique le bras de liaison ou l'amorce conjugée au bras de liaison, ledit conjugué étant ultérieurement couplé au support solide selon l'invention.

Le fragment polymère, notamment homopolynucléotide ou analogue de polynucléotides, peut être un fragment ramifié, c'est-à-dire comportant plusieurs branches. Ce type de fragment peut être obtenu en utilisant un monomère pouvant servir de base à la condensation avec plusieurs monomères sur le même synthon. On cite en particulier le synthon phosphoramidite suivant (Ref. 5250-1 de Clontech) : 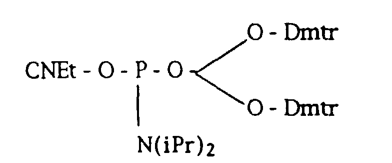 qui peut servir dans le procédé de synthèse du type synthèse automatique d'ADN et peut se condenser avec deux monomères en parallèle.

Avantageusement, ledit polymère et en particulier le fragment homopolynucléotidique ou analogue de polynucléotide intercalé entre le support solide et l'amorce comporte à son extrémité qui n'est pas liée à l'amorce, un groupe fonctionnel terminal réactif pouvant établir un lien covalent avec le support solide. On cite en particulier comme dit groupe fonctionnel terminal réactif du fragment polymère, un groupe hydroxyl ou une amine et de préférence encore un groupe phosphate.

L'utilisation d'un groupement phosphate terminal est particulièrement recommandée lorsque le bras de liaison est un poly-T. En l'absence de groupe phosphate, un bras de liaison poly-A ou poly-C est plus efficace.

Les méthodes de synthèse automatique d'ADN sur support solide permettent de phosphoryler chimiquement l'extrémité 5' terminale de quantités importantes d'oligonucléotides. On peut donc par ces méthodes préparer des bras de liaison de type homopolynucléotides ou analogues de polynucléotides ou des conjugués de ces bras de liaison et de l'amorce, phosphorylés à l'extrémité 5' du bras de liaison.

Selon l'invention, le support solide est constitué de polymère organique ou inorganique fonctionalisé.

Selon la présente invention certaines caractéristiques du support solide sont avantageuses pour pouvoir allonger une amorce oligonucléotidique fixée à un support solide en un produit d'amplification. Tout d'abord, ce support solide doit être thermo-résistant, c'est-à-dire capable de résister aux fortes températures d'une PCR (100°C) et doit avoir une bonne conduction thermique. Il doit être fonctionalisé, c'est-à-dire comporter des fonctions chimiques, afin de permettre la fixation stable d'une amorce oligonucléotidique sur le support solide. Cette liaison amorce-support solide doit être également résistante aux fortes températures. C'est pourquoi selon la présente invention, une liaison covalente est préférée. La Taq polymérase ou autre enzyme responsable de l'élongation ne doit pas être inhibée par des composants du support. Enfin, un tel support doit avoir des propriétés optiques permettant une détection colorimétrique ou fluorescente sans bruit de fond. Un support transparent est préféré car il permet l'utilisation de tous les types de lecteurs.

On utilise donc de préférence des types de plastique qui possèdent une grande résistance aux fortes températures. On cite en particulier les matières plastiques, notamment à base de polystyrène modifié thermo-résistant, de copolymère styrène-acrylonitrile, de polycarbonate, de polypropylène ou de verre.

On peut utiliser des supports plastiques solides fonctionalisés par traitement UV pour induire l'apparition de groupes fonctionnels NH₂ (ref. 1). Toutefois, selon la présente invention, le support solide peut être un support polyfonctionalisé c'est-à-dire comportant une multiplicité de groupes fonctionnels, notamment aldéhyde, carboxyl, amine, hydroxyl ou thiol qui favorisent l'établissement d'un lien covalent stable avec le bras de liaison consistant dans ladite molécule polyfonctionnelle liée à l'amorce.

De façon appropriée, on utilise des supports plastiques qui ont été traités par traitement corona ou irradiation gamma pour induire l'apparition d'une multiplicité de groupes fonctionnels. Ce type de traitement est aisé et évite l'utilisation de réactifs chimiques de fonctionalisation.

Comme support thermo-résistant préféré on utilise en particulier du polycarbonate ou un copolymère de styrène-acrylonitrile fonctionalisés entre autres par traitement corona ou irradiation gamma.

Des méthodes de fixation covalente d'oligonucléotides sur des supports solides fonctionalisés par l'intermédiaire d'un groupe fonctionnel terminal de l'oligonucléotide sont connues (Réf. 1 à 5). Toutefois, selon l'invention, les fragments homopolynucléotidiques peuvent se fixer sur le support solide par les groupes fonctionnels naturels, notamment amine et hydroxyl des bases nucléotidiques elles-mêmes, ou par un groupe fonctionnel terminal notamment phosphate.

De même les fragments de type analogue de polynucléotides peuvent aussi se fixer sur le support solide par un groupe terminal ou l'un des groupes fonctionnels, notamment amine ou hydroxyle substitués sur les monomères.

Ce couplage entre les groupes fonctionnels du bras de liaison, notamment du fragment homopolynucléotidique ou analogue de polynucléotides, et les groupes fonctionnels du support peut se faire par couplage chimique en présence d'agents d'activation conventionnels. En particulier on réalise un couplage chimique à un support polyfonctionnel en présence d'un agent d'activation de type carbodiimide, telle que l'EDC.

La présente invention a également pour objet un procédé d'immobilisation sur un support solide thermo-résistant fonctionnalisé d'une amorce utile dans la mise en oeuvre d'un procédé d'amplification selon la présente invention, caractérisé en ce que l'on réalise un couplage covalent entre ledit support fonctionalisé et un bras de liaison consistant en une molécule polyfonctionnelle liée à l'extrémité 5' de ladite amorce.

Le support solide peut être la surface intérieure du récipient de réaction de PCR, ou un élément solide que l'on introduit dans le récipient avant la réaction, tel que des billes. On cite en particulier comme support solide les surfaces intérieures des puits de microplaques de microtitrage ou des tubes de dosages.

Le format préféré est celui de la microplaque. En effet, sa large utilisation et tous les appareils existant déjà autour de ce format en permettent rapidement et aisément l'automatisation. Deux types de microplaque sont couramment utilisés. Le premier est de type standard avec des cupules cylindriques à fond plat. Le second comporte non pas des cupules cylindriques, comme précédemment, mais des puits en forme de petits tubes tronqués à fond plat. Cette forme de tube permet de s'adapter facilement au cycleur thermique et permet une excellente conduction thermique.

La présente invention a également pour objet un procédé d'immobilisation sur phase solide d'une amorce utile dans la mise en oeuvre d'un procédé d'amplification selon la présente invention, caractérisé en ce que l'on réalise un couplage covalent entre ledit support fonctionalisé et ladite molécule polyfonctionnelle liée à l'extrémité 5' de ladite amorce.

L'amplification en phase solide permet d'effectuer l'amplification et la détection sur le même support. La détection du produit d'amplification allongé sur la surface solide peut se faire de différentes façons. Le double brin fixé au support peut être détecté soit par la mise en évidence d'un marqueur incorporé lors de la PCR, notamment par l'intermédiaire d'une deuxième amorce marquée, soit par l'utilisation d'un agent intercalant de type bromure d'éthidium, YOYO, TOTO, POPO (Molecular Probes Réf. 6 à 8). Après dénaturation, le simple brin spécifiquement fixé au support solide peut être également détecté par une sonde marquée.

La détection consiste par exemple en l'hybridation d'une sonde oligonucléotidique biotinylée qui reconnait spécifiquement le produit d'amplification allongé sur la plaque. Un conjugué Streptavidine-Alkaline Phosphatase reconnait l'entité biotine de la sonde hybridée et génère après déphosphorylation d'un substrat, un produit colorimétrique ou fluorescent.

Le mode de fixation de l'amorce selon la présente invention est déterminant pour l'élongation. En effet, si celle-ci possède un bras de liaison selon l'invention, le rendement d'élongation peut être augmenté de 15 fois suivant le type de bras, alors que le taux de fixation demeure constant quelque soit le bras de liaison.

La quantité d'amorce présente en solution dans la réaction d'amplification joue elle aussi un rôle important pour le rendement d'élongation. Afin d'avoir un rendement optimal d'élongation de l'amorce fixée, une PCR déséquilibrée est utilisée ( Réf. 9). Lors de la PCR, l'amorce fixée au support solide (amorce A) est également présente en solution mais en quantité inférieure, notamment 8 à 16 fois moins importante que l'autre amorce d'amplification (amorce X). L'amplification s'effectue en deux étapes ; l'amplification est d'abord exponentielle jusqu'à ce que l'amorce A en solution soit épuisée, les amorces A fixées au support solide sont ensuite allongées, l'amplification devient alors arithmétique. La première étape permet d'avoir un nombre de copies important, et permet ainsi une élongation sur le support solide plus efficace. La quantité d'amorce mis en solution est critique car elle détermine à quel moment l'amplification arithmétique commence. Une quantité de l'ordre de 5 à 10 pmole pour l'amorce X et de 8 à 16 fois moins pour l'amorce A donne de bons rendements d'élongation.

Le procédé d'amplification selon la présente invention apporte une réelle amélioration par rapport aux techniques habituelles de diagnostic utilisant la PCR, que ce soit pour les maladies infectieuses ou génétiques. Par ailleurs, le procédé selon l'invention permettant d'attacher spécifiquement un seul brin à la phase solide, il peut être aussi intéressant pour le séquençage, spécialement lorsque de grosses quantités doivent être séquencées et qu'une simplification des protocoles et une automatisation s'avèrent indispensables.

D'autres caractéristiques et avantages de la présente invention apparaîtront à la lumière de l'exemple détaillé de réalisation qui va suivre.

La figure 1 représente les différentes étapes de la PCR en phase solide.
X― : amorce X en solution
A― : amorce A en solution
X=======A : produit d'amplification
vvvA― : amorce A fixée

La figure 2 représente le rendement de fixation en pM de l'amorce en fonction de différents bras de liaison.

La figure 3 représente les taux d'élongation en unité de fluorescence en fonction de différents bras de liaison.

La figure 4 représente le rendement d'élongation en fMole d'antisonde hybridée.

La figure 5 représente l'influence de la taille du bras de liaison sur le taux d'élongation.

La figure 6 représente l'influence de différentes fonctions chimiques du bras de liaison sur le taux d'élongation.

Le procédé selon l'invention a été appliqué sur un modèle HLA-DRB (Réf. 10 et 11). Des clones de M13 comportant un insert de 280 pb correspondant au produit d'amplification ont été utilisés comme cible. Ce modèle HLA-DRB a permis de mettre en évidence les paramètres importants intervenant dans l'élongation et d'étudier tout particulièrement le problème de spécificité de cette technique.

### 1) Exemples de différents bras de liaison utilisés.

| | | | |
|---|---|---|---|
| A, | A-Ph | | |
| A-5T, | A-10T | A-20T | A-30T |
| A-5T-Ph, | A-10T-Ph | | |
| A-5A, | A-10A | | |
| A-5A-Ph, | A-10A-Ph | | |
| A-5C, | A-10C | | |
| A-5C-Ph, | A-10C-Ph | | |

A = amorce fixée : CCCCACAGCACGTTTC(T,C)TG
Ph = Groupement phosphate 5' terminal
xT, xA, xC = queue en position 5' terminal de x base T, de x base A ou de x base C respectivement.

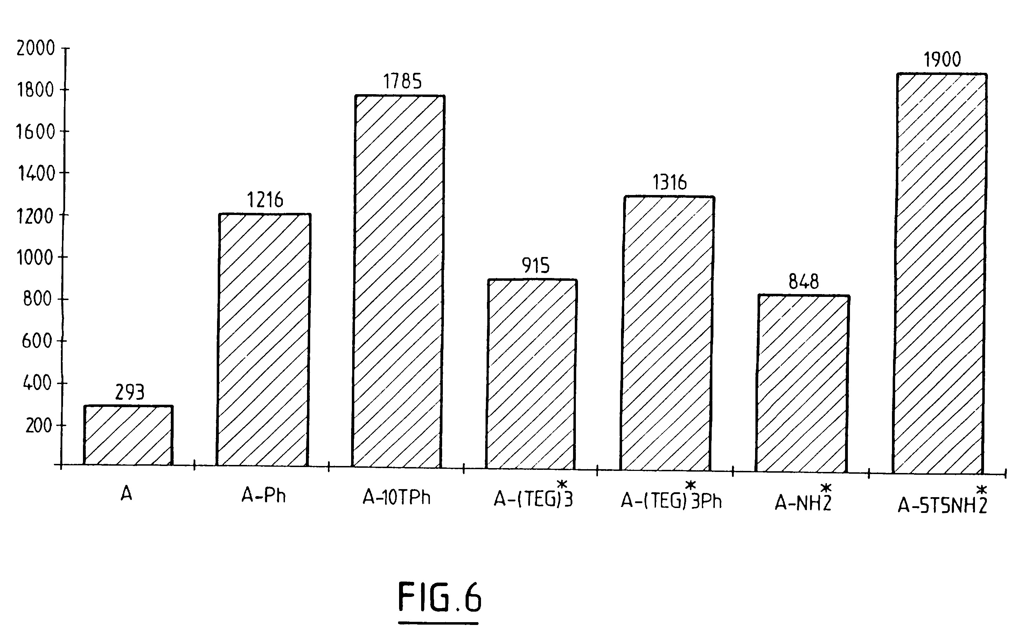

On a aussi utilisé (figure 6) les bras de liaison comprenant des fragments polymères du type analogues de polynucléotides :
A - NH₂*
A - 5T - 5 NH₂*
A - (TEG)₃*
A - (TEG)₃* Ph

Le bras - NH₂* correspond ici à un bras alkyl aminé introduit via le synthon Unilink® Amino Modifier de Clontech de formule

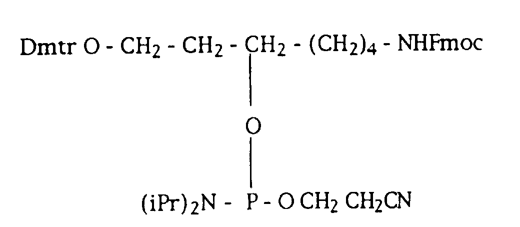

Le bras - 5T - 5 NH₂* correspond à la condensation successive de 5 nucléotides T puis 5 synthons Unilink® dans une synthèse du type phosphoramidite.

La condensation de synthons Unilink® conduit à un fragment analogue de polynucléotide avec des restes alkylamines greffés sur les monomères.
- (TEG)₃ ^{*} correspond à la condensation du synthon de Glen Research (Ref : 10 -1909 x) de formule : 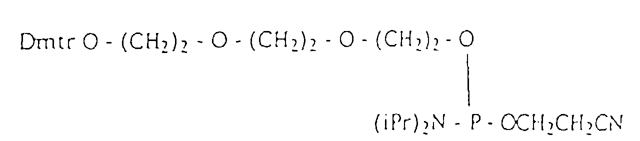
- (TEG)₃ ^{*} - Ph est obtenu par une phosphorylation en 5' terminale du conjugué A - (TEG)₃ ^{*} selon la synthèse phosphoramidite.

### 2) Support solide

Deux types de support thermo-résistants polyfonctionalisés fournis par la société Nunc, ont été utilisés pour effectuer l'évaluation des différents bras de liaison. Le type I est un polystyrène modifié thermo-résistant et le type Il est un copolymère styréne-acrylonitril. Ils ont un format de microplaque et sont mis à cycler dans un thermocycleur fournis par Nunc.

Ces supports plastiques ont été fonctionalisés par un traitement Corona ou irradiation gamme, qui consiste de façon classique à envoyer des décharges électriques dans une enceinte à atmosphère contrôlée et à pression fixe. Cette fonctionalisation permet de rendre le support plastique capable de fixer des amorces oligonucléotidiques. Ce traitement fait apparaitre, à la surface du plastique, des groupements fonctionnels de type amine, alcool, aldéhyde, cétone, acide carboxylique, thiol... qui réagissent chimiquement avec l'oligonucléotide pour former une liaison stable. Les types de liaisons formées, quoique très thermorésistantes, sont toutefois pour l'instant mal connues.

On réalise le couplage chimique en présence d'un agent activateur selon le protocole suivant.

10 à 100 pmole d'oligonucléotides sont mis à fixer par puits en présence d'éthyl carbodiimide (EDC) 10 à 50mM final et de N-Méthyl Imidazole 10 à 50mM final pH 7, dans un volume final de 100 µl. Les plaques sont incubées 5 à 15 heures à 50°C puis lavées 4 fois avec une solution 0,4 N NaOH et 0,25 % tween® 20 chauffées à 50°C.

### 3) Elongation de l'amorce sur la phase solide

L'élongation se fait dans un volume final de 50 µl par puits. L'ADN cible 15 à 100 ng est amplifié dans un mélange comprenant du tampon 1X PCR Buffer II (Perkin Elmer), 0,25 mM MgCl² (Sigma), 200 µM de dATP, dCTP, dGTP, dTTP (Pharmacia), 80 ng d'amorce X (CCGCTGCACTGTGAAGCTCT) et 10ng d'amorce A avec ou sans linker et 1,2 unité de Taq polymérase (Perkin Elmer). L'amorce X se trouve en solution uniquement alors que l'amorce A est fixée au support solide et en solution. L'amplification se fait sur un thermocycleur adapté au format microplaque en utilisant la méthode suivante :
cycle 1:
   5 min à 94°C
cycle 2 à 30 :
   30 s à 94°C
   30 s à 55°C
   30 s à 72°C
cycle 31 :
   5 min à 72°C
   4°C

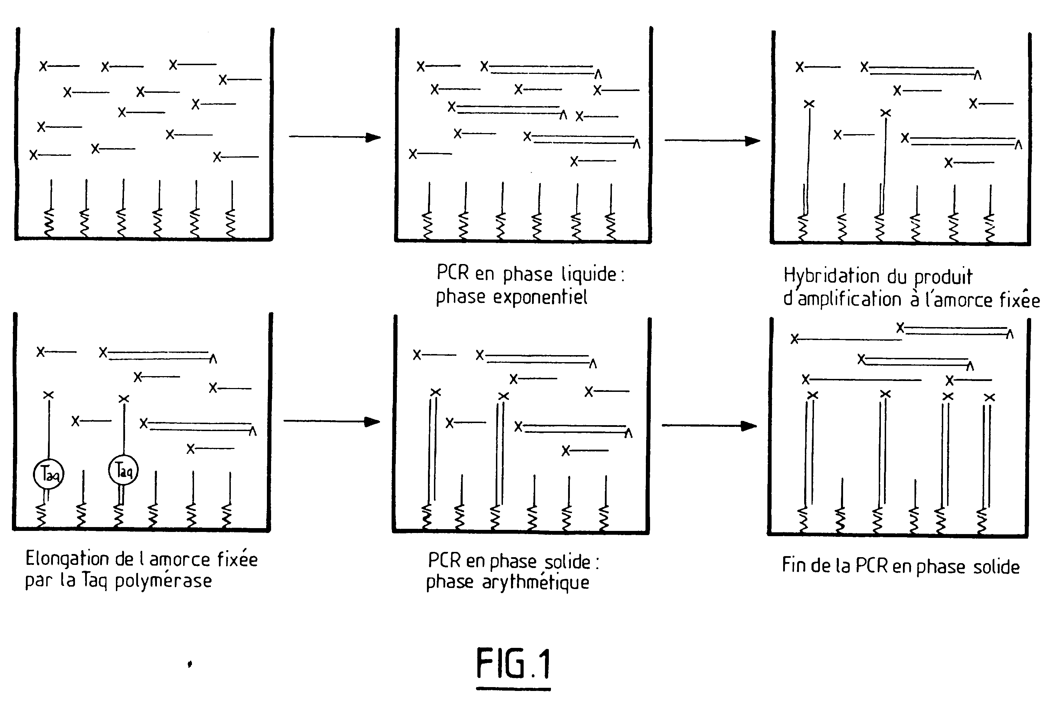

La figure 1 représente les différentes étapes de la PCR en phase solide. Après amplification, les produits d'amplification allongés sur la phase solide sont dénaturés. Les puits sont vidés puis lavés 3 fois avec de la soude O,4M pendant 10 min.

### 4) Détection

Deux types de détection ont été utilisés. Une détection enzymatique semi-quantitative et une détection radioactive quantitative.

La détection enzymatique se réalise comme décrit dans la littérature (9). Dans cette méthode, après dénaturation 1pM de sonde biotinylée complémentaire à une région du brin allongé sur le support solide est hybridée.

L'hybride est révélée par un conjugué streptavidine - alkalyne phosphatase qui transforme un substrat en un produit colorimètrique fluorescent ou chimiluminescent.

Pour la détection radioactive, une sonde marquée au P³² est hybridée sur le brin allongé au support solide et la radioactivité est comptée dans un compteur β.

### 5) Résultats

La capacité de fixation de l'amorce sur le support solide a été quantifiée par fixation d'amorces kinasés radioactivement en 5' et a permis de montrer qui le type 1 fixait de l'ordre de 1 pM d'amorce par puits, tandis que le type II fixait 0,2 pM.

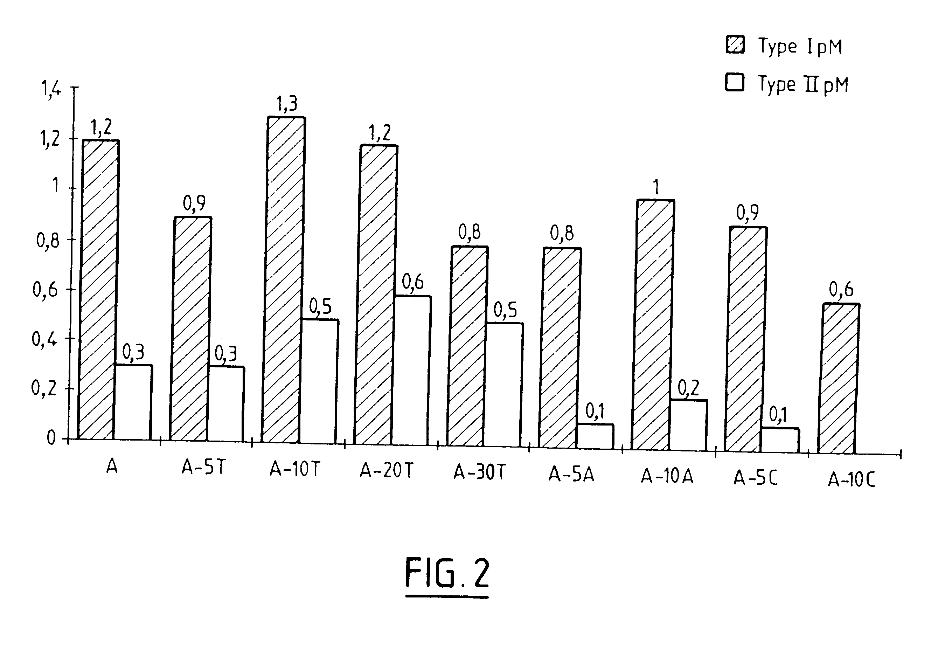

La figure 2 représente la capacité de fixation de l'amorce aux deux supports microplaque en fonction des différents types de bras de liaison. Pour un type de support donné, la capacité de fixation ne varie pas de façon significative en fonction des bras utilisés.

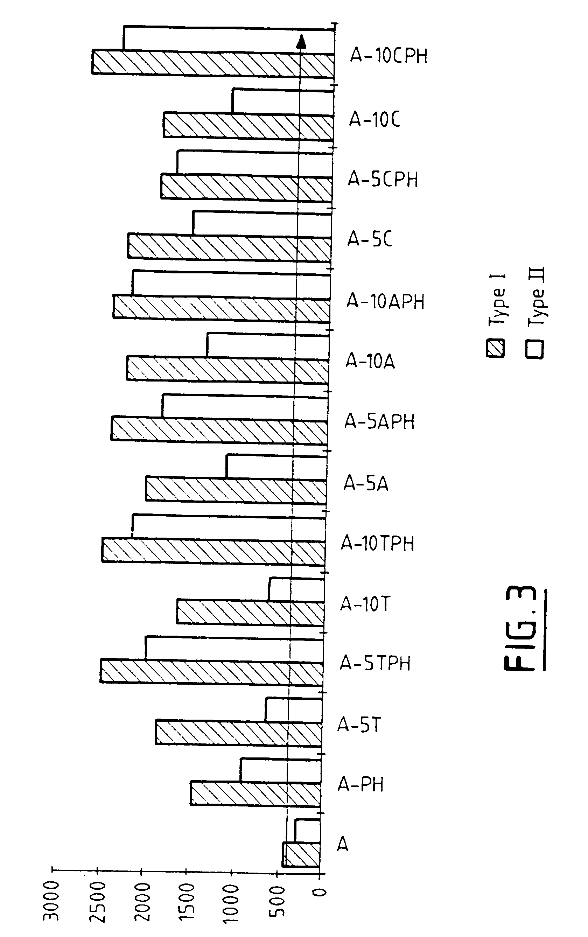

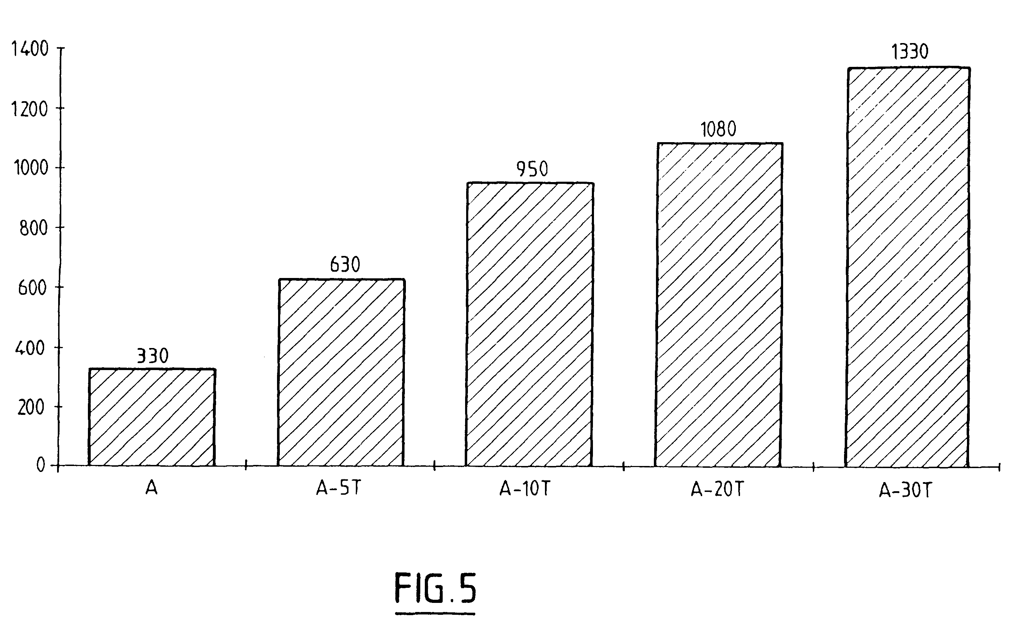

Par contre, le rendement d'élongation varie énormément en fonction du bras de liaison employé. Les résultats sont présentés aux figures 3 et 5 qui représentent les taux d'élongation des supports en fonction des différents bras utilisés. L'influence du bras attaché à l'amorce est déterminante pour le rendement d'élongation qui peut être augmenté de 15 fois suivant le type de linker utilisé, le taux de fixation demeurant constant.

Les fragments poly-A et poly-C sont plus efficaces que les poly-T. En effet, pour des bras de liaison de même taille ils donnent, pour l'élongation, des signaux plus forts. L'ajout d'un groupement phosphate améliore toujours le rendement d'élongation et cette augmentation est plus sensible avec les bras de liaison poly-T qu'avec les bras de liaison poly-A ou poly-C.

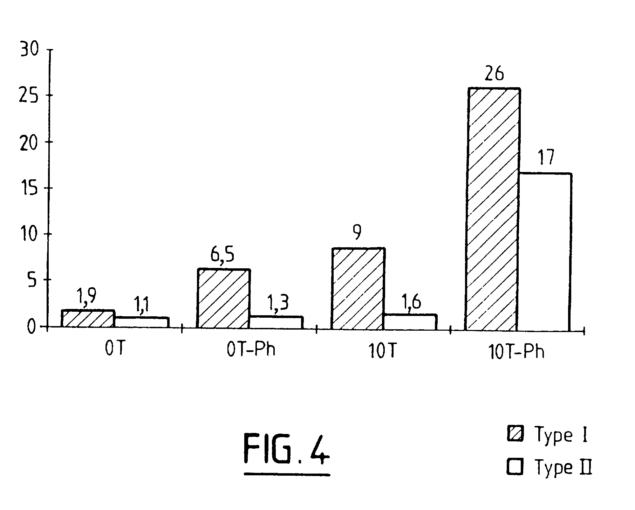

L'élongation a été quantifiée indirectement par hybridation de sondes radioactives complémentaires à la partie allongée par PCR. Le taux d'hybridation a été vérifié en hybridant des sondes radioactives à des puits ayant une quantité connue d'amorces fixées. Le taux d'élongation varie suivant le type de bras de liaison utilisé, entre 2fM et 38fM ou entre 1fM et 17 fM pour type I et type II, respectivement. Ces résultats sont présentés à la figure 4.

La présence de plusieurs groupes fonctionnels au sein du bras de liaison permet d'augmenter le taux d'élongation de l'amorce fixée au support solide. Ainsi comme le montre la Figure 6 on remarque une augmentation de 45 % lorsqu'on intercale 10T entre l'amorce et un groupement phosphate terminal. De même, le fait de rajouter une fonction phosphate à un bras de liaison (TEG)₃ ^{*} permet d'augmenter le signal de 40 % et de 350% par rapport à l'amorce seule sans bras de liaison. Le même phénomène est constaté lorsqu'un bras 5T5NH₂ ^{*} est utilisé; le taux d'élongation est augmenté de 110 % par rapport à un bras NH₂ ^{*}. L'utilisation de plusieurs fonctions chimiques différentes permet donc de favoriser l'élongation de l'amorce sur la phase solide en augmentant les types de liaisons terminales.

La quantité d'oligonucléotides allongées est en fait très faible par rapport à la quantité fixée sur le support. Malgré cette faible quantité, on parvient à des rapports signal sur bruit de l'ordre de 30 avec la technique de détection enzymatique décrite ce qui est tout à fait suffisant pour une application diagnostique.

### BIBLIOGRAPHIE

**1.** "Covalent immobilization DNA onto Polystyrene microwells : the molecules are only bound at the 5' end" . S.R. Rasmussen, et al. Anal. Biochem. 198, 138-142, (1991).
**2.** "A sensitive nonisotopic hybridization assay for HIV-1 DNA". G. H. KELLER, et al. Anal. Biochem. 177, 27-32 (1989).
**3.** "Immobilization of DNA via oligonucleotides containing an aldehyde or carboxylic acid group at the 5' terminus". J.N. Kremsky et al. Nucleic Acid Res. Vol. 15, Num.7, 2891-2909, (1987).
**4.** "Detection of human immunodeficiency virus type 1 RNA in plasma from high risk pediatric patients by using the self sustained sequence replication reaction". C.E Bush, J. Clin. Microbiol. 30, 281-286, (1992).
**5.** "Genetic analysis of amplified DNA with immobilized sequence-specific oligonucleotide probes". R.K. Saiki, et al. pNAS 86, 6230-6234 (1989).
**6.** "Use of the fluorescent dye YOYO-1 to quantifie oligonucleotides immobilized on plastic plates". M. Ogura, et al. Biotechniques 16, 1032 (1994).
**7.** "Stable dye-DNA intercalaton complexes as reagents for high-sensitivity fluorescent detection". A.N. Glazer, H.S. Rye, Nature 359, 859 (1992).
**8.** "Fluorometric assay using dimeric dyes for double- and single-stranded DNA and RNA with picogram sensitivity." H.S. Rye, et al. Anal. Biochem. 208, 144 (1993).
**9.** "Combined polymerase chain reaction-hybridization microplate assay used for detection of bovine leukemia virus and salmonella". S.R. Rasmussen, et al. Clin. Chem., 40:200-5 (1994).
**10.** "Structure, sequence and polymorphism in the HLA-D region". J. Trowsdale, et al. Immuno. Rev., 1985, 85: 5-43.
**11.** "HLA class II nucleotide sequences, 1992." J.G. Bodmer. Eur. J. Immunogen., 1993, 20:47-79.

## Revendications

1. Procédé d'amplification d'acides nucléiques en phase solide dans lequel on utilise une amorce immobilisée sur un support solide thermo-résistant fonctionnalisé, caractérisé en ce que ladite amorce est immobilisée sur le support solide par l'intermédiaire d'un bras de liaison consistant en une molécule polyfonctionnelle établissant un lien covalent entre le support solide et un premier groupe fonctionnel de ladite molécule polyfonctionnelle, et entre l'extrémité 5' de ladite amorce et un deuxième groupe fonctionnel de ladite molécule polyfonctionnelle.

2. Procédé selon la revendication 1, caractérisé en ce que ladite molécule polyfonctionnelle comprend un fragment polymère.

3. Procédé selon la revendication 2, caractérisé en ce que chaque unité monomérique dudit fragment polymère comporte au moins un groupe fonctionnel.

4. Procédé selon l'une des revendications 2 ou 3, caractérisé en ce que ledit fragment polymère comporte au moins 5, de préférence au moins 10, monomères.

5. Procédé selon l'une des revendications 1 à 4, caractérisé en ce que lesdits groupes fonctionnels de la molécule, notamment du fragment polymère sont choisis parmi les groupes amine, hydroxyl, carboxyl, aldehyde, thiol ou phosphate.

6. Procédé selon la revendication 5, caractérisé en ce que lesdits groupes fonctionels sont choisis parmi les groupes amine, hydroxyl ou phosphate.

7. Procédé selon l'une des revendications 1 à 4, caractérisé en ce que ladite molécule polyfonctionnelle comprend un fragment polymère de type homopolynucléotide ou un fragment d'un analogue de polynucléotide.

8. Procédé selon la revendication 7, caractérisé en ce que le fragment polymère est un homopolynucléotide comportant entre 10 et 50 nucléotides.

9. Procédé selon l'une des revendications 1 à 8, caractérisé en ce que ladite molécule polyfonctionnelle comprend un fragment polymère analogue de polynucléotide de formule : 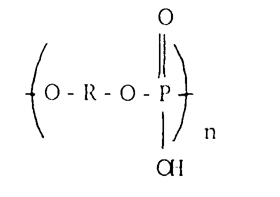 où R est un reste aliphatique portant au moins un groupe fonctionnel et n est un entier de 2 à 50.

10. Procédé selon la revendicatoin 9, caractérisé en ce que ledit fragment polymère analogue de polynucléotide comporte plusieurs groupes amines ou hydroxyl greffés sur chaque monomère.

11. Procédé selon l'une des revendications 2 à 10, caractérisé en ce que le fragment polymère comporte un groupe phosphate terminal à son extrémité qui n'est pas liée à l'amorce.

12. Procédé selon l'une des revendications 1 à 11, caractérisé en ce que le support solide est constitué d'un polymère organique ou inorganique.

13. Procédé selon la revendication 12, caractérisé en ce que le support solide est un matériau plastique thermo-résistant fonctionnalisé par traitement corona ou irradiation gamma.

14. Procédé selon l'une des revendications 1 à 13, caractérisé en ce que le support solide est constitué par du polystyrène modifié thermo-résistant tel qu'un copolymère styrène-acrylonitrile ou du polycarbonate.

15. Procédé selon l'une des revendications 1 à 13, caractérisé en ce que l'amorce fixée au support solide est également présente en solution, mais en quantité moins importante que l'autre amorce en solution lors de la réaction d'amplification.

16. Procédé selon la revendication 15, caractérisé en ce que l'amorce fixée au support solide est également présente en solution en quantité 8 à 16 fois inférieure à celle de l'autre amorce en solution.

17. Procédé selon l'une des revendications 1 à 16, caractérisé en ce que le support solide est constitué par la surface intérieure du récipient dans lequel on effectue l'amplification.

18. Trousse de réactifs pour la mise en oeuvre d'un procédé d'amplification selon l'une des revendications 1 à 17, caractérisée en ce qu'elle comporte un support solide thermo-résistant fonctionnalisé sur lequel est immobilisée une amorce par l'intermédiaire d'un lien covalent avec une molécule polyfonctionnelle qui notamment comprend un fragment polymère.

19. Procédé d'immobilisation sur un support solide thermo-résistant fonctionnalisés d'une amorce utile dans la mise en oeuvre d'un procédé d'amplification selon l'une des revendications 1 à 17, caractérisé en ce que l'on réalise un couplage covalent entre ledit support fonctionnalisé et un bras de liaison consistant en une molécule polyfonctionnelle liée à l'extrémité 5' de ladite amorce.

## Patentansprüche

1. Verfahren zur Amplifikation von Nucleinsäuren in Festphase unter Verwendung eines auf einem thermoresistenten, funktionalisierten, festen Träger immobilisierten Primers, dadurch gekennzeichnet, daß der Primer auf dem festen Träger immobilisiert ist über ein Verbindungsglied, das aus einem polyfunktionellen Molekül besteht, welches eine kovalente Bindung zwischen dem festen Träger und einer ersten funktionellen Gruppe des polyfunktionellen Moleküls und zwischen dem 5'-Terminus des Primers und einer zweiten funktionellen Gruppe des polyfunktionellen Moleküls ausbildet.

2. Verfahren nach Anspruch 1, dadurch gekennzeichnet, daß das polyfunktionelle Molekül ein Polymerfragment umfaßt.

3. Verfahren nach Anspruch 2, dadurch gekennzeichnet, daß jede Monomereinheit des Polymerfragments mindestens eine funktionelle Gruppe umfaßt.

4. Verfahren nach einem der Ansprüche 2 oder 3, dadurch gekennzeichnet, daß das Polymerfragment mindestens 5, vorzugsweise mindestens 10 Monomere umfaßt.

5. Verfahren nach einem der Ansprüche 1 bis 4, dadurch gekennzeichnet, daß die funktionellen Gruppen des Moleküls, insbesondere des Polymerfragments ausgewählt sind aus Amin-, Hydroxyl-, Carboxyl-, Aldehyd-, Thiol- oder Phosphatgruppen.

6. Verfahren nach Anspruch 5, dadurch gekennzeichnet, daß die funktionellen Gruppen ausgewählt sind aus Amin-, Hydroxyl- oder Phosphatgruppen.

7. Verfahren nach einem der Ansprüche 1 bis 4, dadurch gekennzeichnet, daß das polyfunktionelle Molekül ein Polymerfragment vom Typ Homopolynucleotid oder ein Fragment eines Polynucleotidanalogen umfaßt.

8. Verfahren nach Anspruch 7, dadurch gekennzeichnet, daß das Polymerfragment ein Homopolynucleotid ist, das 10 bis 50 Nucleotide umfaßt.

9. Verfahren nach einem der Ansprüche 1 bis 8, dadurch gekennzeichnet, daß das polyfunktionelle Molekül ein Polynucleotidanalog-Polymerfragment umfaßt mit der Formel: 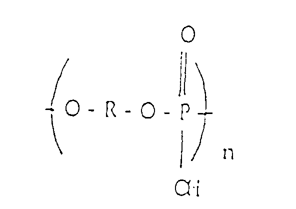 worin R ein aliphatischer Rest ist, der mindestens eine funktionelle Gruppe trägt und n eine ganze Zahl von 2 bis 50 ist.

10. Verfahren nach Anspruch 9, dadurch gekennzeichnet, daß das Polynucleotidanalog-Polymerfragment mehrere Amin- oder Hydroxylgruppen, gepfropft auf jedes Monomere, umfaßt.

11. Verfahren nach einem der Ansprüche 2 bis 10, dadurch gekennzeichnet, daß das Polymerfragment eine endständige Phosphatgruppe an dem Terminus, der nicht mit dem Primer verbunden ist, umfaßt.

12. Verfahren nach einem der Ansprüche 1 bis 11, dadurch gekennzeichnet, daß der feste Träger aus einem organischen oder anorganischen Polymer gebildet ist.

13. Verfahren nach Anspruch 12, dadurch gekennzeichnet, daß der feste Träger ein plastisches thermoresistentes Material, funktionalisiert durch Corona-Behandlung oder Gamma-Bestrahlung, ist.

14. Verfahren nach einem der Ansprüche 1 bis 13, dadurch gekennzeichnet, daß der feste Träger aus thermoresistentem modifiziertem Polystyrol, wie einem Styrol-Acrylnitril-Copolymeren oder Polycarbonat, besteht.

15. Verfahren nach einem der Ansprüche 1 bis 13, dadurch gekennzeichnet, daß der an den festen Träger fixierte Primer auch in Lösung vorhanden ist, jedoch in einer weniger bedeutenden Menge als der andere Primer in Lösung während der Amplifikationsreaktion.

16. Verfahren nach Anspruch 15, dadurch gekennzeichnet, daß der an dem festen Träger fixierte Primer ebenfalls in Lösung vorhanden ist in einer Menge von 8- bis 16-fach unter der des anderen in Lösung befindlichen Primers.

17. Verfahren nach einem der Ansprüche 1 bis 16, dadurch gekennzeichnet, daß der feste Träger aus einer inneren Oberfläche innerhalb des Gefäßes, in dem die Amplifikation durchgeführt wird, gebildet wird.

18. Reagentiensatz zur Durchführung eines Amplifikationsverfahrens nach einem der Ansprüche 1 bis 17, dadurch gekennzeichnet, daß er einen festen, thermoresistenten, funktionalisierten Träger umfaßt, auf dem ein Primer über eine kovalente Bindung mit einem polyfunktionellen Molekül, das insbesondere ein Polymerfragment umfaßt, immobilisiert ist.

19. Verfahren zur Immobilisierung eines Primers, der zur Durchführung des Amplifikationsverfahrens nach einem der Ansprüche 1 bis 17 geeignet ist, an einem festen, thermoresisienten, funktionalisierten Träger, dadurch gekennzeichnet, daß man eine kovalente Kupplung zwischen dem funktionalisierten Träger und einem Verbindungsglied, das aus einem polyfunktionellen Molekül, gebunden an den 5'-Terminus des Primers, besteht, durchführt.

## Claims

1. Process for the solid-phase amplification of nucleic acids, in which a primer immobilized on a functionalized heat-resistant solid support is used, characterized in that the said primer is immobilized on the solid support by means of a linker arm consisting of a polyfunctional molecule which establishes a covalent bond between the solid support and a first functional group of the said polyfunctional molecule, and between the 5' end of the said primer and a second functional group of the said polyfunctional molecule.

2. Process according to Claim 1, characterized in that the said polyfunctional molecule comprises a polymer fragment.

3. Process according to Claim 2, characterized in that each monomer unit of the said polymer fragment contains at least one functional group.

4. Process according to either of Claims 2 and 3, characterized in that the said polymer fragment contains at least 5 monomers and preferably at least 10 monomers.

5. Process according to one of Claims 1 to 4, characterized in that the said functional groups of the molecule, in particular of the polymer fragment, are chosen from amine, hydroxyl, carboxyl, aldehyde, thiol and phosphate groups.

6. Process according to Claim 5, characterized in that the said functional groups are chosen from amine, hydroxyl and phosphate groups.

7. Process according to one of Claims 1 to 4, characterized in that the said polyfunctional molecule comprises a polymer fragment of homopolynucleotide type or a fragment of a polynucleotide analogue.

8. Process according to Claim 7, characterized in that the polymer fragment is a homopolynucleotide containing between 10 and 50 nucleotides.

9. Process according to one of Claims 1 to 8, characterized in that the said polyfunctional molecule comprises a polynucleotide-analogue polymer fragment of formula: 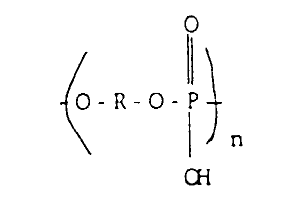 where R is an aliphatic residue bearing at least one functional group and n is an integer from 2 to 50.

10. Process according to Claim 9, characterized in that the said polynucleotide-analogue polymer fragment contains several amine or hydroxyl groups grafted onto each monomer.

11. Process according to one of Claims 2 to 10, characterized in that the polymer fragment contains a terminal phosphate group at its end which is not linked to the primer.

12. Process according to one of Claims 1 to 11, characterized in that the solid support consists of an organic or inorganic polymer.

13. Process according to Claim 12, characterized in that the solid support is a heat-resistant plastic material functionalized by corona treatment or gamma-irradiation.

14. Process according to one of Claims 1 to 13, characterized in that the solid support consists of heat-resistant modified polystyrene such as a styrene/acrylonitrile copolymer or polycarbonate.

15. Process according to one of Claims 1 to 13, characterized in that the primer bound to the solid support is also present in solution, but in lower amount than the other primer in solution during the amplification reaction.

16. Process according to Claim 15, characterized in that the primer bound to the solid support is also present in solution in an amount 8 to 16 times less than that of the other primer in solution.

17. Process according to one of Claims 1 to 16, characterized in that the solid support consists of the inner surface of the container in which the amplification is carried out.

18. Reagent kit for carrying out an amplification process according to one of Claims 1 to 17, characterized in that it contains a functionalized heat-resistant solid support on which a primer is immobilized by means of a covalent bond with a polyfunctional molecule, which in particular comprises a polymer fragment.

19. Process for the immobilization of a primer on a functionalized heat-resistant solid support, this primer being useful in carrying out an amplification process according to one of Claims 1 to 17, characterized in that covalent coupling is carried out between the said functionalized support and a linker arm consisting of a polyfunctional molecule linked to the 5' end of the said primer.
